# EUROPEAN PATENT APPLICATION

(11) **EP 2 039 392 A1**
(43) Date of publication of application: **25.03.2009**
(21) Application number: 06843964.5
(22) Date of filing: 13.11.2006
(51) Int. Cl.: A61N 5/06, A61N 5/067, A61N 2/00, A61B 5/0476, A61B 5/0484, A61M 21/00, A61B 5/048

(54) **DEVICE FOR PHYSIOTHERAPEUTICALLY TREATING DISEASES OF DIFFERENT ETHIOLOGIES**

(30) Priority: 26.06.2006 RU 2006122570
(71) Applicant: Zakrytoe Aktsionernoe Obschestvo "Yaninvest", Moscow 123060 (RU)
(72) Inventor: KAPLAN, Aleksandr Yakovlevich, Moscow, 117639 (RU); MISHANIN, Nikolai Vasilevich, Moscow, 117405 (RU); SHISHKINA, Anna Aleksandrovna, Moscow, 105043 (RU)
(74) Representative: Andrae, Steffen
(86) International application number: PCT/RU2006/000592
(87) International publication number: WO 2008/002183

(57) **Abstract**

The present invention is aimed at improving efficiency of therapeutic action. The above technical effect is achieved in a device for physiotherapeutic treatment of diseases of various etiology comprising a control unit 1 connected to a color action unit 30, an intracavitary action unit 28 embodied in the form of a rectal and/or vaginal probe within which a laser and a source of magnetic field and/or a catheter are located, an epicutaneous action unit 29 embodied in the form of an epicutaneous electrode, and/or an epicutaneous radiator embodied in the form of a case containing sources of magnetic field and color action; said device being provided with a means 31 for recording electroencephalogram with a unit 32 for dividing thereof into three frequency ranges with a separate output for each range, each output being connected through the control unit 1 to a separate monochromatic radiation former of the color action unit 30. Moreover, the unit 32 for dividing into three frequency ranges is made with frequency ranges that correspond to alpha-, beta- and theta-rhythms of brain, and the color action unit 30 is embodied in the form of a TV screen or computer monitor.

## Description

### FIELD OF THE INVENTION

The present invention relates to medical engineering, in particular to devices for physiotherapy, and may be used for treating diseases of various etiology.

### BACKGROUND OF THE INVENTION

There has been known a device for physiotherapy of prostate diseases comprising a control unit, an ultrasound source, electrophoresis and electrostimulation units connected to rectal and urethral probes-electrodes (see, USSR Inventor's Certificate No. 1,803,148, A 61 N 1/00, 1993 /1/). The known device, when in use under complex application conditions, provides a combined action by ultrasonic oscillations with medicinal phono-electrophoresis and electrostimulation of pathology areas. The drawback with the known device resides in the absence of a unit correcting a curative action based on the change in a patient condition, thus reducing efficiency of the treatment.

There has been known a device for treating patients with chronic prostatitis complicated by a sexual function disorder (see, RU Patent No. 2,008,043, A 61 N 5/06, 1994 /2/). The device comprises an electrostimulation unit with an elastic transurethral catheter-electrode, two electrodes one of which being made in the form of transurethral catheter or plate, a laser action unit comprising an infrared semiconductor laser. Moreover, the device is provided with a control unit connected to means for evaluating a patient's state. Said means for evaluating a patient's state are made in the form of a photosensor and acoustic pulse sensor to record contraction of smooth muscles. Based on signals received from the sensors, the control unit corrects conditions of curative effect. The drawback with the known device resides in its low therapeutic efficiency. This is due to a comparatively low information content of a biological feedback to be ensured by the above mentioned sensors. For instance, the pulse sensor somewhat reflects the general state of a patient, but fails to serve a criterion for efficiency of an action upon prostate. The sensor to record contraction of smooth muscles affords individual selection of frequency and current intensity of electrostimulation, but fails to determine a required action time and may not be used as a criterion of improvements in a functional state of prostate. Besides, change in a pulse rate and smooth muscle contraction may be caused by a number of factors, apart from therapeutic action. This results in such correction of action conditions that may result not in the enhancement of therapeutic action efficiency but in its reduction.

The closest prior art device with respect to the claimed invention as to its technical essence and a result to be achieved is disclosed in a device for physiotherapeutic treatment of diseases of urogenital system, which is known from RU Patent No. 2,124,911, A 61 N 5/06, 1999 /3/. The known device comprises a control unit connected to a color therapy (color action) unit, intracavitary and epicutaneous electrostimulation units, two laser action (intracavitary and epicutaneous) units and electrodes, one being embodied in the form of a rectal probe with its interior designed to accommodate a laser and a permanent magnet (for intracavitary action), an epicutaneous electrode, and an epicutaneous radiator embodied in the form of a case within which a magnet, light-emitting diodes and a continuous wave laser (for epicutaneous action) are located.

The drawback with the known device resides in its insufficiently high therapeutic efficiency due to the fact that it fails to comprise means enabling one to optimize action factors used in therapy, in particular a color action.

### SUMMARY OF THE INVENTION

The present invention disclosing a device for physiotherapeutic treatment of diseases of various etiology is aimed at improving efficiency of therapeutic action.

The above technical effect is achieved in a device for physiotherapeutic treatment of diseases of various etiology comprising a control unit connected to a color action unit, an intracavitary action unit embodied in the form of a rectal and/or vaginal probe within which a laser and a source of magnetic field and/or a catheter are located, an epicutaneous action unit embodied in the form of an epicutaneous electrode, and/or an epicutaneous radiator embodied in the form of a case containing sources of magnetic field and color action; said device being provided with a means for recording electroencephalogram with a unit for dividing thereof into three frequency ranges with a separate output for each range, each output being connected through the control unit to a separate monochromatic radiation former of the color action unit.

The above technical effect is also achieved by the fact that a unit for dividing into three frequency ranges is made with frequency ranges that correspond to alpha-, beta- and theta-rhythms of brain.

The above technical effect is also achieved by the fact that the color action unit is embodied in the form of a TV screen or computer monitor.

The above technical effect is also achieved by the fact that the color action unit is embodied in the form of with multicolor light-emitting diodes located within a visual field of said spectacles.

As is known from /2/ and /3/, simultaneous action of several medical factors on a patient provides synergetic effect, namely an enhanced efficiency of conducting physiotherapeutic procedures, in particular provision of simultaneous intracavitary action using a rectal and/or vaginal probe within which a laser and a source of magnetic field are located, and an external action using an epicutaneous radiator within which a magnet and a light source are located in the form of a light-emitting diode and a continuous wave laser, thus considerably improving efficiency of treating diseases of pelvic organs.

Provision of a device for physiotherapeutic treatment with an epicutaneous radiator having a pulsed laser which is principally used for setting upon perineum, and supplementation of an intracavitary electrostimulation unit with yet another electrode embodied in the form of a transurethral catheter with a laser located on the inside thereof, affords considerable increase in the efficiency of physiotherapeutic action, since action on, for example prostate, is carried out not only with a rectal probe but also with a transurethral catheter, under conditions of simultaneous stimulation of biologically active points associated with prostate. In doing so, the transurethral catheter may be used for physiotherapeutic action not only on pelvic organs but also on other organs, for example for treating diseases of ear, throat and nose. In these cases the catheter may be used both individually and in combination with the epicutaneous radiator and the color action unit.

A prior art color action unit, when used in combination with an intracavitary action unit and/or an epicutaneous (external) action unit, enhances achievement of therapeutic effect. However, said prior art color action unit operates by an algorithm to be assigned by a doctor, that is intensity of color action, color saturation and radiation spectrum itself are "imposed upon" a patient and sometimes may cause discomfort with a patient, rejection of certain colors, which naturally affects the results of treatment. To avoid such negative factor, it is recommended that a patient himself, consciously or unconsciously, may choose optimal parameters of color action, that is spectrum, intensity, color saturation.

The claimed device is aimed at solving this problem. With this aim in view, a device is provided with a means for recording electroencephalogram with a unit for dividing thereof into three frequency ranges with a separate output for each range, each output being connected through the control unit to a separate monochromatic radiation former of a color action unit.

Accomplishment of a unit for dividing into three frequency ranges with frequency ranges that correspond to alpha-, beta- and theta-rhythms of brain, is determined by the fact that they correspond to a generally accepted (in neurophysiology) breakdown of EEG into main rhythms in a wakeful state of the human organism.

When the proposed device is used in therapeutic procedures, a signal at the output of a means for recording electroencephalogram is divided into three frequency ranges and supplied to a color action unit (to monochromatic formers of a color picture, for example to separate cathodes of a color TV electron gun or a computer monitor). Accordingly, the screen of a color action unit will produce one or other color picture, which will act on a patient. It is established experimentally that a human being may unconsciously choose a color which is comfortable to him, and said color, in virtue of a positive influence on a human being, creates optimal conditions to ensure the greatest effect of therapeutic procedures, whereas a biological feedback circuit "EEG - color gamut - color perception - EEG" realizes said possibility through the control of corresponding monochromatic radiators that create a color gamut on the screen of a monitor under observation of a patient.

### BRIEF DESCRIPTION OF THE DRAWINGS

The essence of the proposed device for physiotherapeutic treatment explained by its embodiments and drawings, in which:

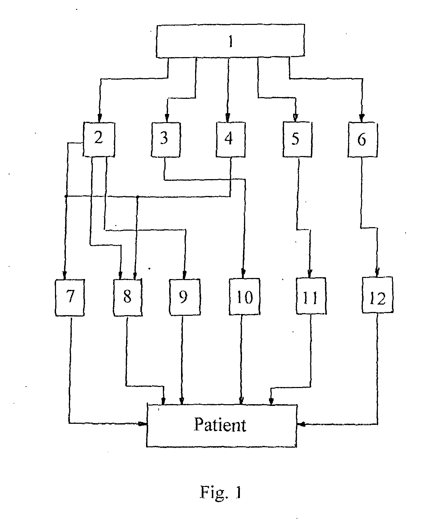

FIG. 1 is a block diagram of a prior art device;

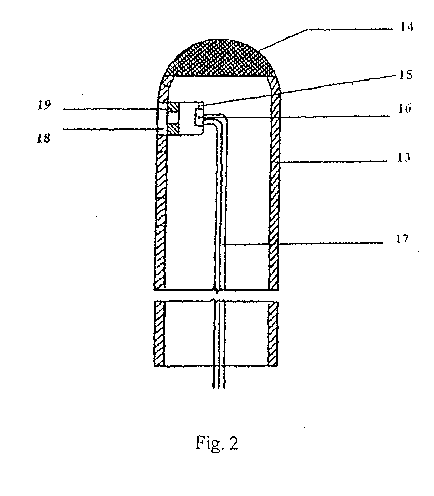

FIG. 2 shows a longitudinal section of a rectal (or vaginal) probe;

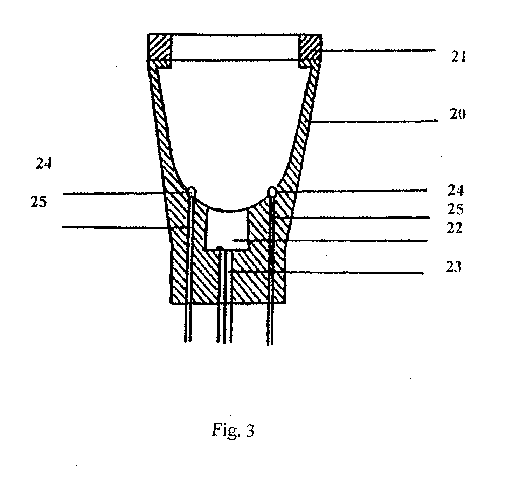

FIG. 3 shows a lateral section of an epicutaneous radiator;

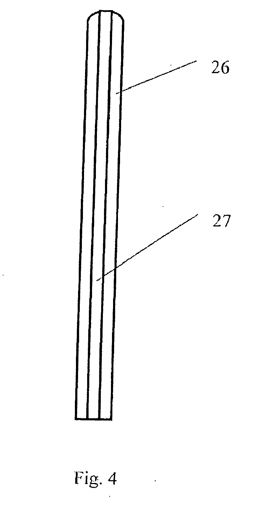

FIG. 4 shows a longitudinal section of a catheter distal end;

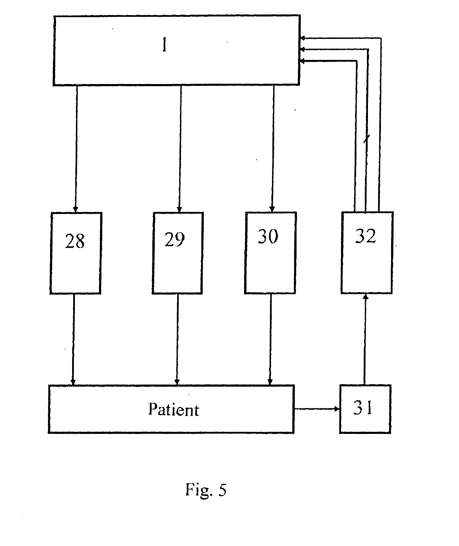

FIG. 5 is a block diagram of a device in accordance with the present invention.

### DETAILED DESCRIPTION OF THE PREFERRED EMBODIMENTS

A prior art device for physiotherapeutic treatment comprises a control unit 1, two electrostimulation units - an intracavitary electrostimulation unit 2 and an epicutaneous electrostimulation unit 3, two laser action units - an intracavitary laser action unit 4 and an epicutaneous laser action unit 5, a color therapy unit 6, a rectal probe 7, a transurethral catheter 8, an electrode for electrostimulation 9, which is embodied in the form of a plate, an epicutaneous electrode 10 with three contacts, two outer of which being connected to each other, i.e. in essence it represents an electrode with two contacts, an epicutaneous radiator with a magnet, a light-emitting diode and a continuous wave laser 11, special spectacles 12 to be put on by a patient when conducting a color therapy session. A device makes possible the realization of various combinations of operation of its units with corresponding terminal devices (a rectal probe, a transurethral catheter, an epicutaneous electrode and radiators, an electrostimulation plate electrode, special spectacles).

The rectal probe comprises a case 13, an electrostimulation contact 14, a radiator body 15 within which an emitting semiconductor crystal 16 with three leads 17 are located, said body being provided with an open output window 18. In the output window 18, there is installed a permanent ring-shaped magnet 19 with its plane being perpendicular to the radiation propagation. An epicutaneous radiator comprises a case 20, a ring-shaped magnet 21, a semiconductor infra-red laser source 22 with leads 23, and also light-emitting diodes 24 with leads 25. A catheter that may be used, as in the prior art device, both in the form of transurethral catheter and for intracavitary action on different organs (ear, throat, nose), comprises a case 26 which at the same time serves as an intracavitary electrostimulation electrode within which a light-emitting diode 27 is located to supply radiation of a semiconductor or helium-neon laser.

The control unit 1 of the proposed device comprises, as in the prior art device, electrostimulation control units - an intracavitary electrostimulation control unit and an epicutaneous electrostimulation control unit, laser action control units - an intracavitary laser action control unit and an epicutaneous laser action control unit, a color therapy control unit 6. The control unit 1 may be embodied in the form of a personal computer provided with relevant software to allow a doctor - operator to replace or adjust a therapeutic action program, thus customizing it for a certain patient. The device comprises corresponding power supplies (not shown in the drawings) which are necessary for operation of all terminal devices to be connected to the control unit to produce intracavitary, epicutaneous and color action. Said terminal devices are clustered (arbitrarily) in three units. An intracavitary action unit 28 may comprise a rectal probe and/or a vaginal probe and/or an intracavitary catheter, which in the prior art device was only used to affect prostate or urethra, whereas in the claimed device it may be used to affect any internal cavities of a patient. An epicutaneous action unit 29 may comprise various epicutaneous electrodes for electrostimulation and/or epicutaneous radiators with magnetic field and color action sources. A color action unit 30 may be embodied in the form of a TV screen or PC monitor or, as in the prior art device, in the form of special spectacles with multicolor light-emitting diodes located within their visual field. In addition, the device comprises an encephalogram recording unit 31 with its output connected to a unit 32 that ensures division of the encephalogram to be recorded into three frequency ranges with a separate output for each range, each output being connected to the control unit.

A device for physiotherapeutic treatment of diseases of various etiology is used as follows. Position of a patient - lying on back in a urologic or gynecologic chair or on a couch. Depending on the chosen treatment conditions and algorithm, the following embodiments are possible within the scope of the present invention.

Embodiment 1. The first electrode embodied in the form of a plate is located under the sacrum of a patient. The second electrode - a transurethral catheter which is a part of the electrostimulation unit 2 and processed with a sterile sea-buckthorn oil or wild-rose oil is passed over urethra up to the boundary of prostatic section of urethra.

Embodiment 2. A catheter described above is used as the first electrode, whereas the second electrode is used in the form of a rectal probe 11 processed with a sterile oil, which is inserted into rectum.

Embodiment 3. A rectal probe inserted into rectum is used as the first electrode, whereas the second electrode is used in the form of a plate, which is located above a pubic region or on perineum.

Embodiment 4*.* A rectal probe inserted into rectum is used as the first electrode, whereas the second electrode is used in the form of a vaginal probe, which is located within vagina.

Embodiment 5. A rectal probe inserted into rectum is used as the first electrode, whereas the second electrode is used in the form of a plate, which is located on perineum, and an epicutaneous radiator, which is located above a pubic region.

Embodiment 6. The first electrode is used in the form of a catheter, which is located within floor of the auricle, whereas the second electrode is used in the form of a plate, which is located on the temple.

Embodiment 7. The first electrode is used in the form of a catheter, which is located within floor of the auricle, whereas the second electrode is used in the form of an epicutaneous radiator, which is located on the temple.

Embodiment 8. The first electrode is used in the form of a catheter, which is inserted into nasopharynx, whereas the second electrode is used in the form of an epicutaneous radiator, which is located above bridge of the nose.

Embodiment 9. The first electrode is used in the form of a catheter, which is inserted into nasopharynx, whereas the second electrode is used in the form of a plate, which is located above bridge of the nose.

The eyes of a patient are covered with special spectacles having multicolor light-emitting diodes to keep out extraneous light action, or in the visual field of a patient there is located a TV or computer monitor connected to the control unit 1.

A doctor makes use of algorithms of action recorded in the computer memory, or on his own sets parameters of therapeutic actions by means of a computer keyboard.

Symbolic information on the chosen conditions of action and a procedure to be performed is presented on a personal computer display (monitor).

To optimize a therapeutic procedure, the effect of main therapeutic factors (electrostimulation and a magnetic field action) is accompanied by a light stimulation in the form of certain color tints to be produced on a TV screen, computer monitor or display spectacles. Color stimulation always begins with one and the same color gamut, which subsequently changes up to stabilization of the most comfortable color gamut under the control action of a patient's electroencephalogram to be recorded by a device 31 and entered through a unit 32 to the control unit and further to a color action unit 30, which is connected to formers of monochromatic radiation (red, blue, green). In such a manner a monitor color gamut gradually change under unconscious action of biological currents of a patient brain until a certain color gamut stabilizes. Precisely this color gamut is then chosen to accompany a therapeutic process, since it was chosen by a patient itself in a biological feedback circuit "EEG - color gamut - color perception - EEG" based on unconscious preference given to a certain color. The possibility of such unconscious selection of a comfortable color gamut was shown in the work by Kaplan A.Y. (see, Kaplan A.Y., Lim J.J., Jin K.S., Park B.W., Byeon J.G., Tarasova S.U. Unconscious operant conditioning in the paradigm of brain-computer interface based on color perception. International Journal of Neuroscience, 2005, Vol. 115, N 6, pp. 781-802.)

When a personal computer is used to control operation of various units of the device, algorithms of action developed by a doctor on his own are kept (memorized) in the computer for multiple use.

The device makes possible the realization of various combinations of its operation with corresponding terminal devices (a rectal probe, a catheter, an epicutaneous electrode and radiators, an electrostimulation plate electrode, special spectacles, etc.).

## Claims

1. A device for physiotherapeutic treatment of diseases of various etiology comprising a control unit (1) connected to a color action unit (30), an intracavitary action unit (28) embodied in the form of a rectal and/ or vaginal probe within which a laser and a source of magnetic field and/or a catheter are located, an epicutaneous action unit (29) embodied in the form of an epicutaneous electrode, and/or an epicutaneous radiator embodied in the form of a case containing sources of magnetic field and color action, **characterized in that** said device is provided with a means (31) for recording electroencephalogram with a unit (32) for dividing thereof into three frequency ranges with a separate output for each range, each output being connected through the control unit (1) to a separate monochromatic radiation former of the color action unit (30).

2. A device for physiotherapeutic treatment of diseases as claimed in Claim 1 **characterized in that** the unit (32) for dividing into three frequency ranges is made with frequency ranges that correspond to alpha-, beta- and theta-rhythms of brain.

3. A device for physiotherapeutic treatment of diseases as claimed in Claim 1 **characterized in that** the color action unit (30) is embodied in the form of a TV screen or computer monitor.

4. A device for physiotherapeutic treatment of diseases as claimed in Claim 1 **characterized in** the color action unit (30) is embodied in the form of spectacles with multicolor light-emitting diodes located within their visual field.
